# EUROPEAN PATENT APPLICATION

(11) **EP 3 100 602 A1**
(43) Date of publication of application: **07.12.2016**
(21) Application number: 14880793.6
(22) Date of filing: 30.01.2014
(51) Int. Cl.: A01C 1/06

(54) **COATING MATERIAL FOR SEEDS AND COATED SEED**

(71) Applicant: Towada Green Tuff Agro-Science Co., Ltd., Tokyo 152-0011 (JP); Kyokuto Pharmaceutical Industrial Co., Ltd., Chuo-ku Tokyo 103-0024 (JP)
(72) Inventor: HIROSE, Yoichiro, Tokyo 105-0012 (JP); TODAKA, Nemuri, Takahagi-shi Ibaraki 318-0004 (JP)
(74) Representative: Novagraaf Technologies
(86) International application number: PCT/JP2014/052118
(87) International publication number: WO 2015/114778

(57) **Abstract**

Provided are: a coating material for seeds that can be obtained by a simple treatment stage; and a coated seed that is coated with the aforesaid coating material, suffers from little physical damage and is almost free from growth inhibition. The coating material for seeds comprises as a major component a pulverized inorganic mineral powder. The coated seed is coated with the aforesaid coating material.

## Description

### Technical Field

The present invention relates to a coating material for seeds obtained through a simple treatment step, and a coated seed.

### Background Art

In order to protect a crop from disease or insect damage, technologies to protect a seed with a pesticide, antagonistic microorganisms, or the like have been proposed.

Patent Literatures 1 to 7 have proposed inventions relating to an antagonistic microorganism coated seed having a high disease-controlling effect and a high storage stability, obtained by inoculating a seed with an antagonistic microorganism under reduced pressure.

Patent Document 8 has proposed an invention relating to a coated seed suitable for protecting a crop from disease or insect damage by coating a seed with a coating material containing an inorganic mineral powder and a thermosetting resin powder.

### Citation List

### Patent Literature

Patent Literature 1: JP 11-4606 A
Patent Literature 2: JP 2002-3322 A
Patent Literature 3: JP 2003-034607 A
Patent Literature 4: JP 2007-77118 A
Patent Literature 5: JP 3-501800 A
Patent Literature 6: US 2,932,128
Patent Literature 7: JP 2011-201800 A
Patent Literature 8: WO 2010/087380 A

### Summary of Invention

### Technical Problem

However, in the above related art, for example, physical damage such as seed deterioration due to a decompression treatment may be caused, or growth of a seed may be hindered by a coating agent containing a special spreading agent, an organic protective agent, or the like disadvantageously.

If a seed can be coated with a coating material for seeds not requiring such a decompression treatment, special spreading agent, organic protective agent, or the like as described above, a coating material for seeds and a coated seed can be obtained through a simpler step.

Therefore, an object of the present invention is to provide a coating material for seeds obtained through a simple treatment step, and a coated seed coated with the coating material, suffering from little physical damage, and almost free from growth inhibition.

### Solution to Problem

In order to solve the above problems, the inventor has found that a pulverized inorganic mineral powder is suitable for a coating material to coat a seed, and has reached the present invention.

That is, an invention according to claim 1 is a coating material for seeds containing a pulverized inorganic mineral powder.

An invention according to claim 2 is the coating material for seeds according to claim 1, in which the inorganic mineral powder has a Mohs hardness of 1 to 7.

An invention according to claim 3 is the coating material for seeds according to claim 2, in which the pulverized inorganic mineral powder has an average particle diameter of 0.9 µm to 100 µm.

An invention according to claim 4 is the coating material for seeds according to claim 3, in which the pulverized inorganic mineral powder has a mode value of 0.25 µm to 1.10 µm in a particle size distribution.

An invention according to claim 5 is a coating material for seeds, in which the coating material for seeds according to claim 4 is a liquid composition having a predetermined viscosity.

An invention according to claim 6 is a coated seed coated with the coating material for seeds according to any one of claims 1 to 5.

### Advantageous Effects of Invention

The present invention can provide a coating material for seeds obtained through a simple treatment step, and a coated seed coated with the coating material, suffering from little physical damage, and almost free from growth inhibition.

### Brief Description of Drawings

Fig. 1 is a diagram illustrating a particle size distribution of Towada stone powder in a suspension after Towada stone powder is suspended in distilled water.
Fig. 2 is a diagram illustrating a particle size distribution of sieved Towada stone powder.
Fig. 3 is a diagram illustrating a particle size distribution of Towada stone powder sieved and then firstly pulverized.
Fig. 4 is a diagram illustrating a particle size distribution of Towada stone powder secondly pulverized.
Fig. 5 is a diagram illustrating a particle size distribution of Towada stone powder thirdly pulverized.
Fig. 6 is a diagram illustrating a particle size distribution of Towada stone powder fourthly pulverized.
Fig. 7 is a diagram illustrating a particle size distribution of Towada stone powder fifthly pulverized.
Fig. 8 is a diagram illustrating an average particle diameter of Towada stone powder, a median diameter thereof, and a standard deviation thereof in the sieving step and the pulverizing step.
Fig. 9 is a diagram illustrating the pH of a suspension in each step of the pulverizing treatment.
Fig. 10 is a diagram illustrating a viscosity of the suspension in each step of the pulverizing treatment (measured at a temperature of 27.5°C).
Fig. 11 is a diagram illustrating a coating amount of Towada stone powder onto seeds in each step of the pulverizing treatment.
Fig. 12 (a) is a diagram illustrating an average particle diameter of Towada stone powder and a median diameter thereof in each step of the pulverizing treatment of a Towada stone powder suspension (26 wt%) in a second tank of an overflow type precipitation container. Fig. 12(b) is a diagram illustrating an average particle diameter of Towada stone powder and a median diameter thereof in each step of the pulverizing treatment of a Towada stone powder suspension (20 wt%) in a third tank of the overflow type precipitation container.

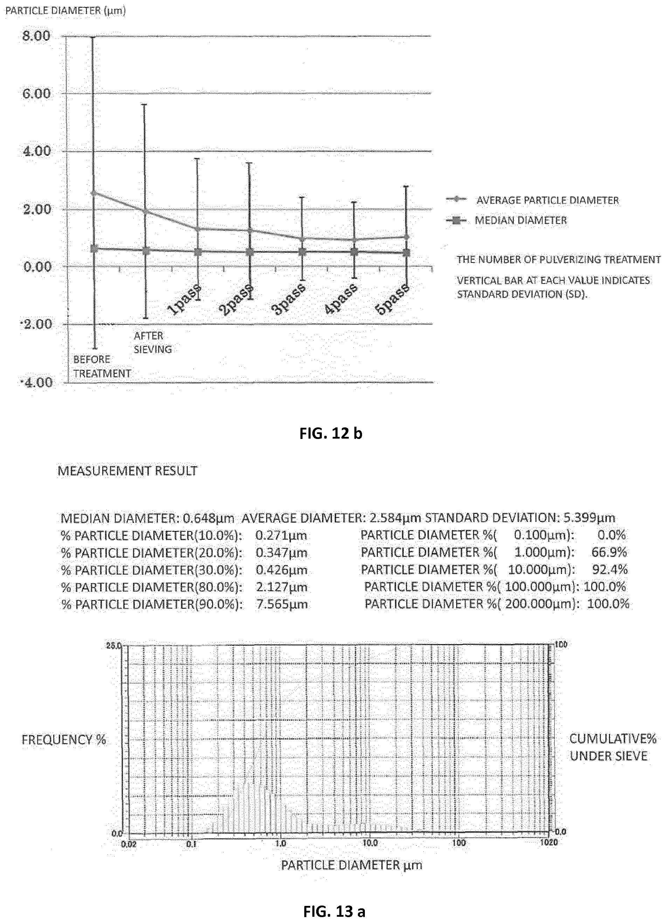

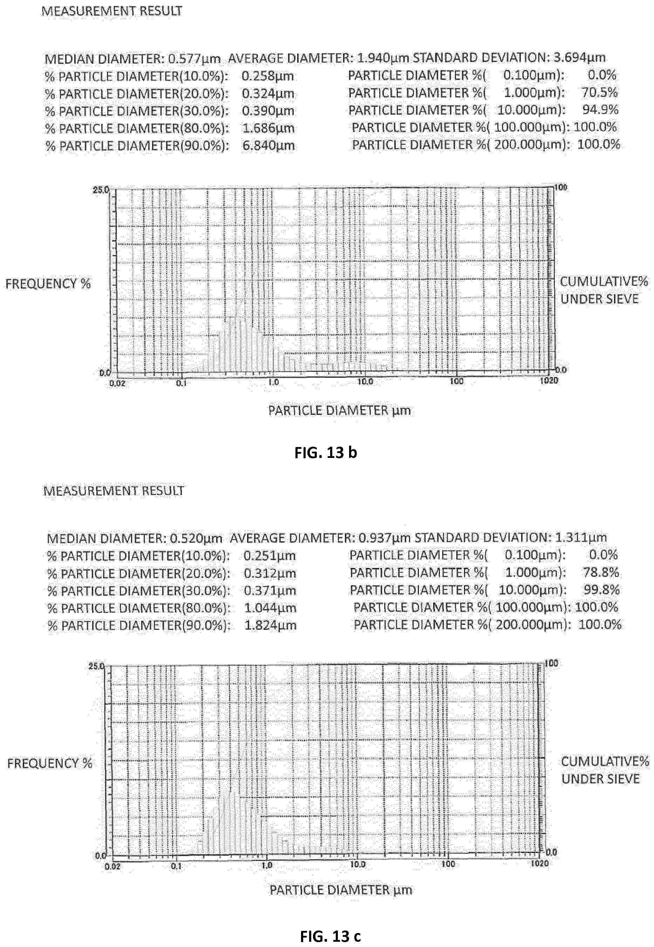

Fig. 13 (a) is a diagram illustrating a particle size distribution of not-pulverized Towada stone powder in a Towada stone powder suspension in the third tank of the overflow type precipitation container. Fig. 13(b) is a diagram illustrating a particle size distribution of sieved Towada stone powder in the Towada stone powder suspension in the third tank of the overflow type precipitation container. Fig. 13(c) is a diagram illustrating a particle size distribution of Towada stone powder pulverized through four stages (4 pass) in the Towada stone powder suspension in the third tank of the overflow type precipitation container.
Fig. 14 is a diagram illustrating a coating amount of Towada stone powder onto seeds of cucumis sativus in each step for pulverizing a Towada stone suspension (20 wt%) in the third tank of the overflow type precipitation container.
Figs. 15 (a) to 15(c) are photographs showing coating states of a Towada stone suspension onto seeds of cucumis sativus. Fig. 15 (a) is a photograph showing a state in a case where powdery Towada stone (DM powder) (20 wt%) is used. Fig. 15(b) is a photograph showing a state in a case where a Towada stone powder suspension (20 wt%, pulverized through four stages) in the third tank of the overflow type precipitation container is used. Fig. 15(c) is a photograph showing a state in a case where water is used as a control.
Fig. 16 is a diagram illustrating a coating amount of an inorganic mineral powder onto seeds of cucumis sativus using a zeolite powder suspension or a bentonite powder suspension.

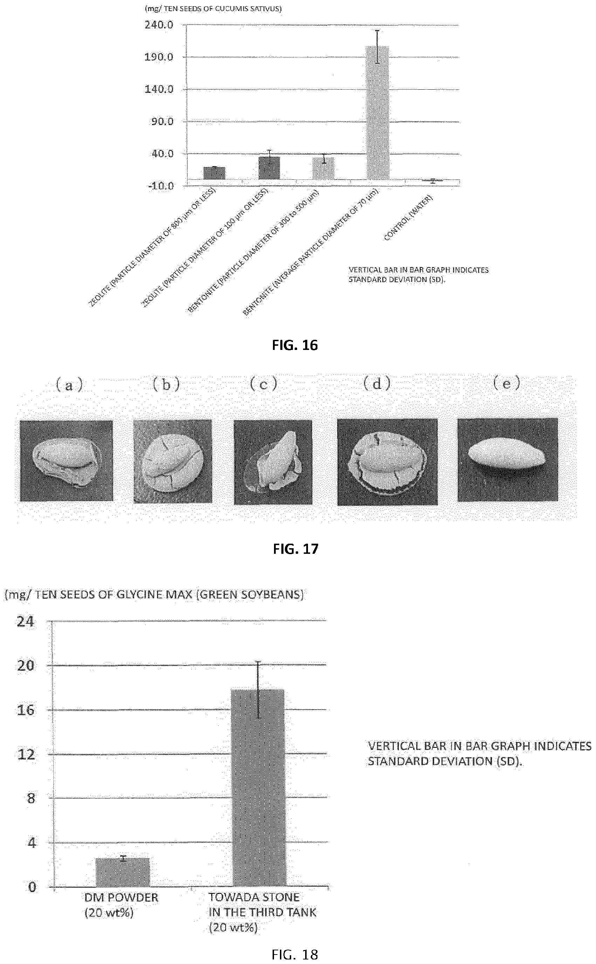

Figs. 17 (a) to 17(e) are photographs showing coating states of an inorganic mineral powder suspension illustrated in Fig. 16 (20 wt%) onto seeds of cucumis sativus. Fig. 17(a) is a photograph showing a state in a case where zeolite having a particle diameter of 800 µm or less is used. Fig. 17(b) is a photograph showing a state in a case where zeolite having a particle diameter of 100 µm or less is used. Fig. 17(c) is a photograph showing a state in a case where bentonite having a particle diameter of 300 µm to 500 µm is used. Fig. 17(d) is a photograph showing a state in a case where bentonite having an average particle diameter of 70 µm is used. Fig. 17(e) is a photograph showing a state in a case where water is used as a control.
Fig. 18 is a diagram illustrating a coating amount of Towada stone powder onto seeds of glycine max (green soybeans).

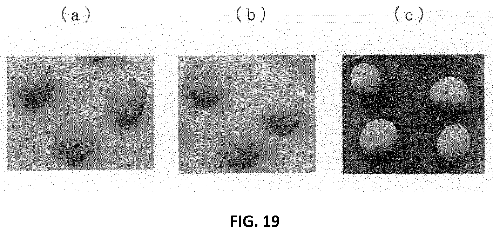

Figs. 19 (a) to 19(c) are photographs showing coating states onto seeds of glycine max (green soybeans) using a Towada stone powder suspension. Fig. 19(a) is a photograph showing a state in a case where powdery Towada stone (DM powder) (20 wt%) is used. Fig. 19 (b) is a photograph showing a state in a case where a Towada stone powder suspension (20 wt%, pulverized through four stages) in the third tank of the overflow type precipitation container is used. Fig. 19 (c) is a photograph showing a state in a case where water is used as a control.

### Description of Embodiments

The present invention relates to a coating material for seeds and a coated seed coated with the coating material.

### 1. Coating material for seeds

The coating material for seeds according to the present invention is a coating material for seeds containing a pulverized inorganic mineral powder. The inorganic mineral includes a natural ore mainly containing silicate secondary minerals, other natural ores, and the like. In this embodiment, a natural ore which is green tuff (official name "dacite pumice tuff") mined in Odate, Hinai, Akita Prefecture and is known under the name "Towada stone" (hereinafter, referred to as "Towada stone"), was used.

Inorganic minerals having different hardnesses, described in exemplary experiments below, were also used.

Towada stone is a complex ore including quartz, albite, chlorite, or the like as a mineral composition, and contains a lot of minerals. Towada stone adsorbs and releases a substance because of porosity, for example, and is also used as an agricultural fertilizer. The main composition of Towada stone is shown in Table 1.

**[Table 1]**

| chemical composition of Towada stone (%) | | | | | | | |
|---|---|---|---|---|---|---|---|
| SiO₂ | Al₂O₅ | Fe₂O₅ | TiO₂ | CaO | MgO | Na₂O | K₂O |
| silicon oxide | aluminum oxide | iron oxide | titanium oxide | calcium oxide | magnesium oxide | sodium oxide | potassium oxide |
| 72.1 | 12.7 | 3.2 | 0.5 | 1.3 | 1.0 | 4.1 | 2.3 |

For pulverizing, it is possible to use a mechanical or manual sieving method, a wet or dry grinding method using a pulverizing device or the like, a method such as natural filtration using a supernatant of a precipitation container, a method of crushing particles with a mechanical force (ball mill, bead mill, or the like), a method of utilizing a grinding force with a media (media) (millstone type grinder or the like), a method of mechanically applying a shock (hammer type grinder or the like), a method of utilizing a shearing force caused by high-speed stirring (stirrer, mixer, or the like), or a method of utilizing a shearing force at the time of passing through a gap at a high pressure (high-pressure homogenizer or the like).

In this embodiment, Towada stone was pulverized using both the wet grinding method and the sieving method, and a coating material for seeds was prepared. Specific treatment steps in pulverizing include the following steps.

### (1) Suspending step

This is a step for obtaining an inorganic mineral powder suspension (hereinafter, referred to as "not-treated suspension") by suspending an inorganic mineral powder in a liquid.

In this embodiment, powdery Towada stone (product name: DMpowder manufactured by Towada Green-tuff AgroScience Co., Ltd.) was suspended in distilled water so as to have a concentration of 30 wt% to obtain a not-treated suspension of Towada stone powder.

### (2) Sieving step

This is a step for sieving the above not-treated suspension to obtain a sieved suspension. The not-treated suspension of Towada stone powder was sieved to obtain a sieved suspension of Towada stone powder.

### (3) Pulverizing step

This is a step for pulverizing the sieved suspension to obtain a pulverized suspension.

In this embodiment, Towada stone powder in the above sieved suspension of Towada stone powder was pulverized using a wet pulverizing device under a high pressure, preferably under 160 MPa or more, to obtain a pulverized suspension of Towada stone powder. This pulverizing step was performed five times.

The following parameters were measured for the Towada stone powder suspension in each step of the above (1) to (3).

### (Particle size distribution)

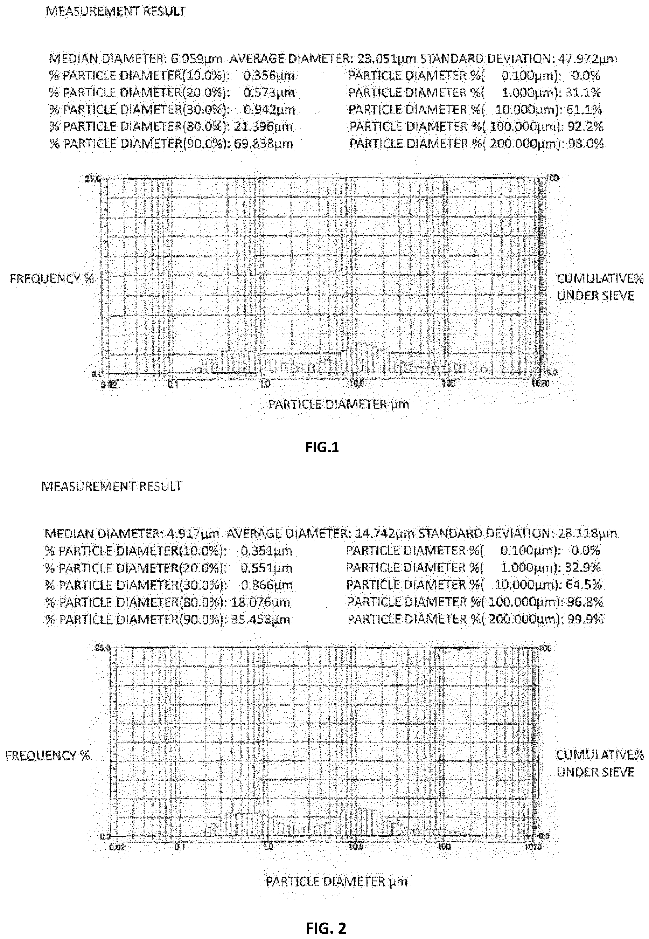

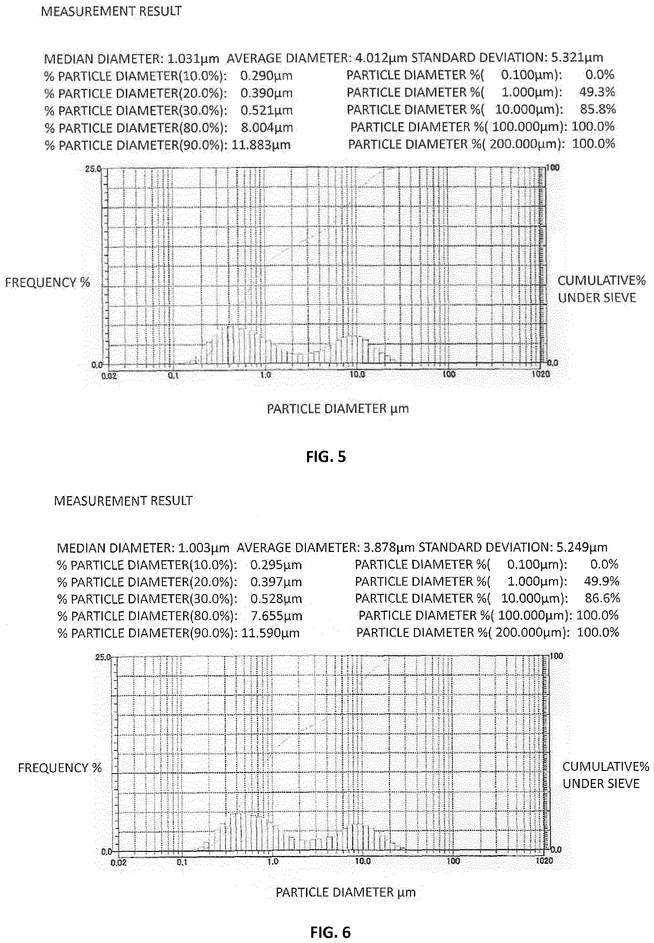

Each of Figs. 1 to 7 is a diagram illustrating a particle size distribution of Towada stone powder in each step of the above (1) to (3).

In the particle size distribution of Towada stone powder in the above not-treated suspension, three distribution peaks were confirmed (Fig. 1).

In the particle size distribution of Towada stone powder in the sieved suspension in the above (2) or in the pulverized suspension in the above (3), it was confirmed that the number of distribution peaks had been reduced to two (Figs. 2 to 7).

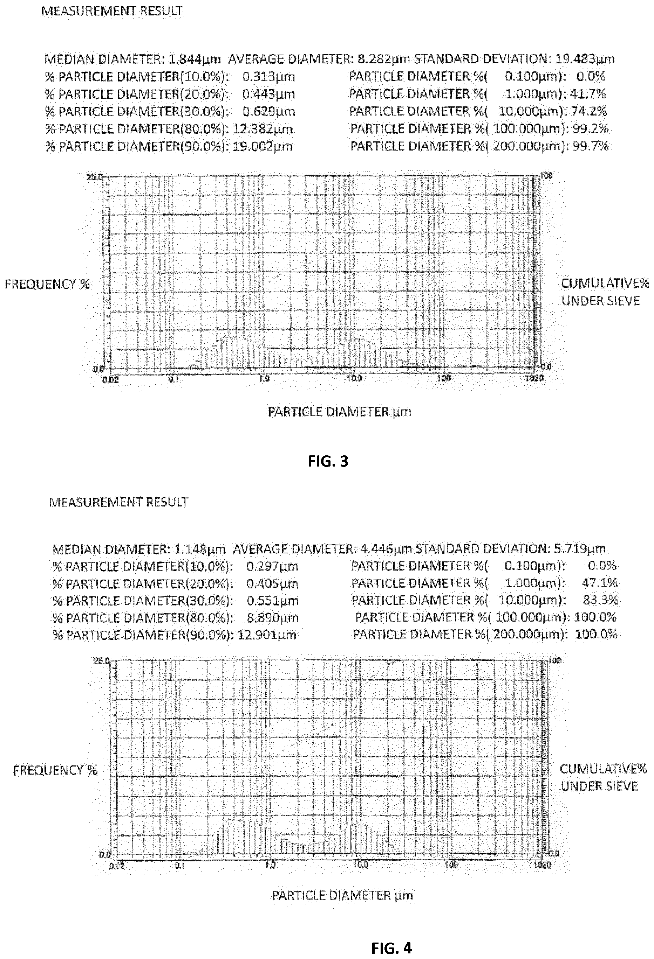

Particularly, due to the first pulverizing treatment in the pulverizing step, the distribution peak around 100 µm disappeared, and only the distribution peak around 0.39 µm and the distribution peak around 11.6 µm appeared among the three distribution peaks confirmed in the particle size distribution in Fig. 1 (Fig. 3).

It was confirmed that the particle size was gradually shifted from the distribution peak around 11.6 µm toward the distribution peak around 0.39 µm (0.25 µm to 1.10 µm) with increase in the number of the pulverizing treatment in the above (3) (Figs. 4 to 7).

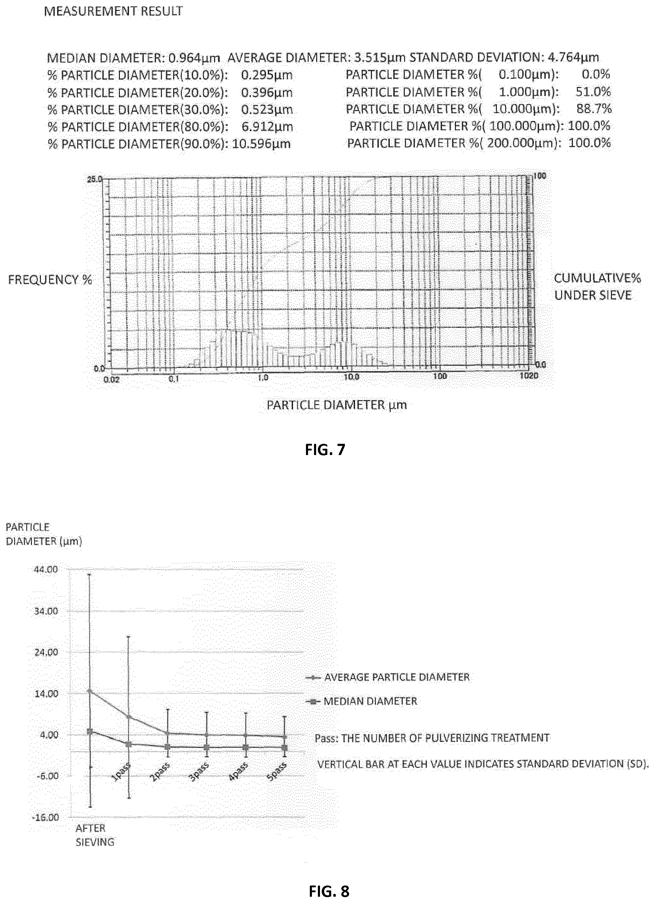

It was confirmed that the average particle diameter of Towada stone powder and the median diameter thereof were reduced with increase in the number of the pulverizing treatment in the above (3), the standard deviation of a particle diameter was reduced, and the average particle diameter tended to be equalized between 3.0 µm to 9.0 µm (Fig. 8).

In this embodiment, the average particle diameter of Towada stone powder tends to be equalized between 3.0 µm to 9.0 µm. However, the range of the average particle diameter can be adjusted by an inorganic mineral powder used, a pulverizing method used, and the like.

### (pH)

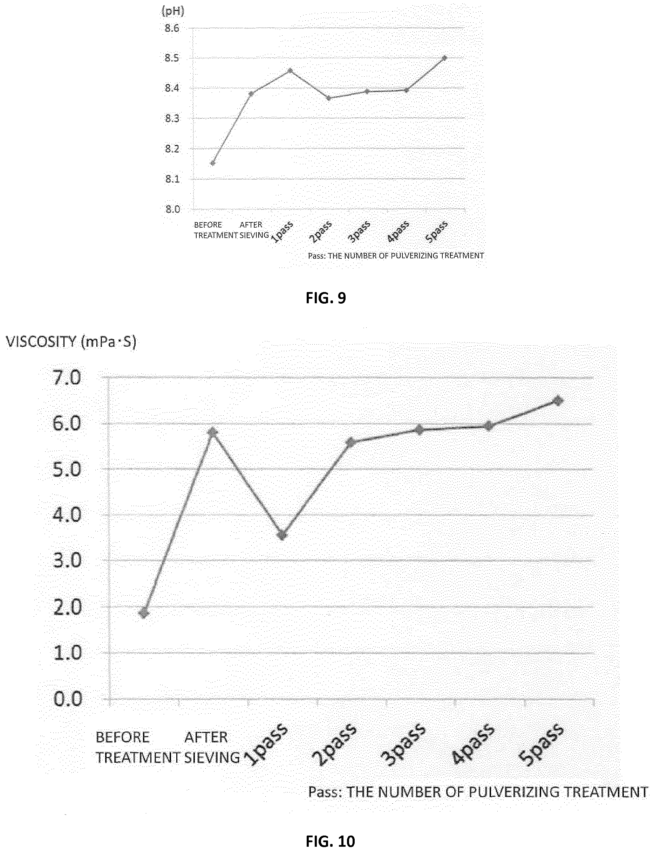

Fig. 9 is a diagram illustrating the pH of the Towada stone powder suspension in each step of the above (1) to (3).

The pH of the sieved suspension was increased about by 0.2 compared to the not-treated suspension. The pH of the pulverized suspension was about 8.4 to 8.5. Therefore, it has been suggested that the weakly alkaline suspension containing pulverized Towada stone powder in a basic state suitable for microorganisms preferably used for agriculture is suitable for a coating material for seeds.

### (Viscosity)

Fig. 10 is a diagram illustrating the viscosity of the Towada stone powder suspension in each step of the above (1) to (3). The viscosity of the suspension was measured at a temperature of 27.5°C.

The viscosity of the sieved suspension was increased about by 3.9 mPa·S compared to the not-treated suspension. Subsequently, the viscosity was reduced temporarily by the first pulverizing treatment. However, thereafter, the viscosity was gradually increased with increase in the number of the pulverizing treatment, and the viscosity was maintained at 5.0 mP·S to 6.5 mP·S.

Therefore, it has been suggested that the suspension containing pulverized Towada stone powder, having a predetermined viscosity suitable for seed coating, is suitable for a coating material for seeds.

In addition, it has been suggested that a suspension containing pulverized inorganic mineral powder other than Towada stone also has a viscosity suitable for a coating material for seeds.

### 2. Coated seed

A coated seed according to the present invention is coated with a coating material for seeds including as a major component a pulverized inorganic mineral powder.

In the present embodiment, seeds of cucumis sativus were used as seeds to be coated. An appropriate amount of the Towada stone powder suspension in each step of the above (1) to (3) was dropwise added to the seeds of cucumis sativus.

Thereafter, the resulting seeds were dried to obtain coated seeds of cucumis sativus coated with Towada stone powder. The coating amount of Towada stone powder in the coated seeds was measured by the following procedures.

Ten seeds of cucumis sativus were weighed (average weight: 270.4 mg, standard deviation: 9.9mg). Thereafter, the Towada stone powder suspension in each step of the above (1) to (3) was dropwise added to the seeds of cucumis sativus at 200 µL/seed.

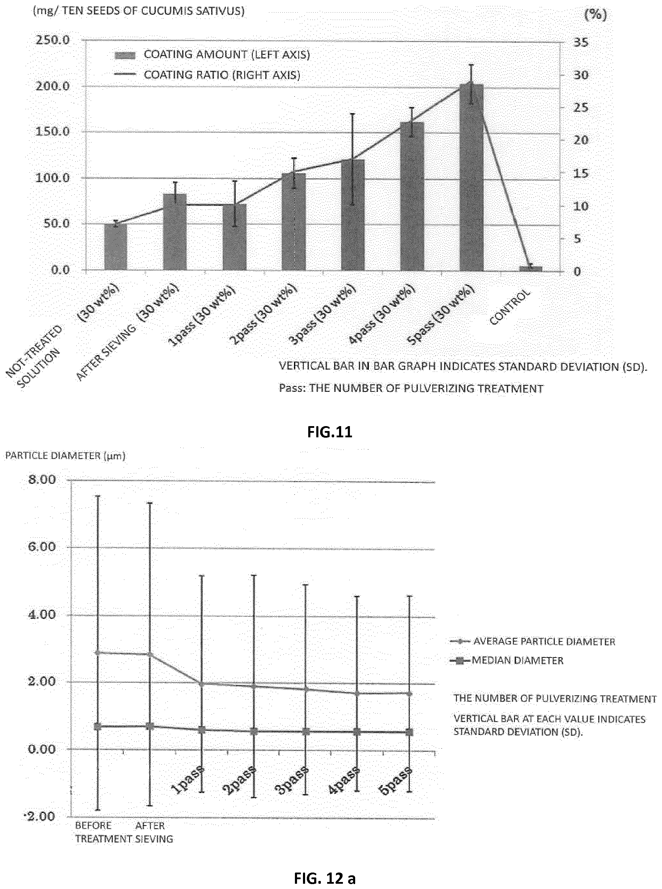

Thereafter, the seeds of cucumis sativus coated with Towada stone powder were naturally dried at room temperature about for 20 hours, and then were weighed. A difference between this weight and the weight of the seeds of cucumis sativus was used as a coating amount of Towada stone powder. Results thereof are illustrated in Fig. 11.

The coating amount of the sieved suspension was slightly larger than the not-treated suspension. It was confirmed that the coating amount onto the seeds of cucumis sativus had been increased with increase in the number of the pulverizing treatment. Particularly, it was confirmed that the coating amount of the suspension fifthly (5 pass) pulverized was about four times that of the not-treated suspension.

According to these results, it was confirmed that the Towada stone powder suspension pulverized such that the average particle diameter of Towada stone powder converged in a range of 3.0 µm to 9.0 µm and the mode value in a particle size distribution converged in a range around 0.39 µm (0.25 µm to 1.10 µm) was suitable for a coating material for seeds.

Particularly, it was confirmed that a weakly alkaline coating material for seeds having a viscosity suitable for seed coating could be prepared only through a step of pulverizing Towada stone powder in a suspension.

In addition, it was confirmed that the coating amount of Towada stone powder in a coating material of a coated seed coated with such a coating material for seeds could be maintained about at 15% to 30%.

Therefore, it is possible to provide a coating material for seeds obtained through a simple treatment step not requiring a spreading agent, an organic protective agent, a decompression treatment, or the like unlike prior art, and a coated seed coated with an appropriate amount of the material, suffering from little physical damage, and almost free from growth inhibition.

In this embodiment, by coating the seeds of cucumis sativus with the suspension fifthly pulverized, the coating ratio of Towada stone was 29% as a maximum value. However, it is expected that the coating ratio depends on the amount of a suspension dropwise added to seeds of cucumis sativus. For reference, the amount of Towada stone powder contained in 2mL of the suspension in each step of the above (1) to (3) is shown in Table 2.

**[Table 2]**

| | amount of microparticles in 2 ml of suspension (mg) |
|---|---|
| not-treated solution | 680.9 |
| after sieving | 827.3 |
| 1 pass (30 wt%) | 711.4 |
| 2 pass (30 wt%) | 709.00 |
| 3 pass (30 wt%) | 704.3 |
| 4 pass (30 wt%) | 710.1 |
| 5 pass (30 wt%) | 711.9 |

In this reference example, the amount of Towada stone powder in the sieved suspension was increased about by 20% compared to the not-treated suspension. Subsequently, the amount of Towada stone powder firstly pulverized was reduced temporarily. However, thereafter, approximately a constant amount of Towada stone powder was maintained with increase in the number of the pulverizing treatment.

These results suggest that a Towada stone powder suspension pulverized such that the average particle diameter of Towada stone powder is equalizedto 4.5 µm or less is suitable for a coating material for seeds when the amount of Towada stone powder in the suspension is constant.

### (Exemplary experiment 1)

An experiment for examining an application of a Towada stone powder suspension filtered in a second tank or a third tank of an overflow type precipitation container including a plurality of tanks to a coating material for seeds was performed.

(1) Measurement of concentration of Towada stone powder suspension in second tank or third tank of precipitation container 1 mL of Towada stone powder suspension filtered in each of a second tank and a third tank of a precipitation container was poured into a glass petri dish, and was weighed.

After measurement of the weight, each suspension was dried by natural drying (14h) and a heat treatment (120°C, 2h), and then was weighed again to calculate the concentration of the suspension (n = 4). The concentration of the Towada stone powder suspension in the second tank was 26 wt%. The concentration of the Towada stone powder suspension in the third tank was 20 wt%.

### (2) Particle size distribution of Towada stone powder suspension in second tank or third tank of precipitation container

The Towada stone powder suspension filtered in each of the second tank and the third tank of a precipitation container was used as the not-treated suspension. The not-treated suspension was sieved according to the above sieving step, and was pulverized according to the above pulverizing step to obtain a sieved suspension and a pulverized suspension. The particle size distribution of Towada stone powder in a suspension subjected to each treatment was measured. Results thereof are illustrated in Fig. 12.

As for the Towada stone powder suspension in the second tank of the precipitation container, it was confirmed that the average particle diameter of Towada powder and the median diameter thereof were reduced with increase in the number of the pulverizing treatment, the standard deviation of a particle diameter was reduced, and the average particle diameter tended to be equalized around 2.0 µm (Fig. 12(a)).

As for the Towada stone powder suspension in the third tank of the precipitation container, it was confirmed that the average particle diameter of Towada stone powder and the median diameter thereof were reduced with increase in the number of the pulverizing treatment, the standard deviation of a particle diameter was reduced, and the average particle diameter tended to be equalized around 1.0 µm (Fig. 12(b)).

In the particle size distribution of the Towada stone powder suspension in the third tank of the precipitation container, only a peak around 0.39 µm (0.25 µm to 1.10 µm) appeared, and the peak around 0.39 µm became significant due to the pulverizing treatment (Fig. 13).

### (3) Coated seed

In this exemplary experiment, seeds of cucumis sativus (product name: Narunarukyuri manufactured by Nanto Seed Co., Ltd.) was used as seeds to be coated. An appropriate amount of the Towada stone powder suspension in the third tank of the precipitation container in each step of the pulverizing treatment was dropwise added to the seeds of cucumissativus. Thereafter, the resulting seeds were dried to obtain coated seeds of cucumis sativus coated with Towada stone powder. The coating amount of Towada stone powder in the coated seeds was measured by the following procedures.

Ten seeds of cucumis sativus were weighed (average weight: 259.1 mg, standard deviation: 10.8 mg). Thereafter, the Towada stone powder suspension (20 wt%) in the third tank of the precipitation container in each step of the pulverizing treatment was dropwise added to the seeds of cucumis sativus at 200 µL/seed.

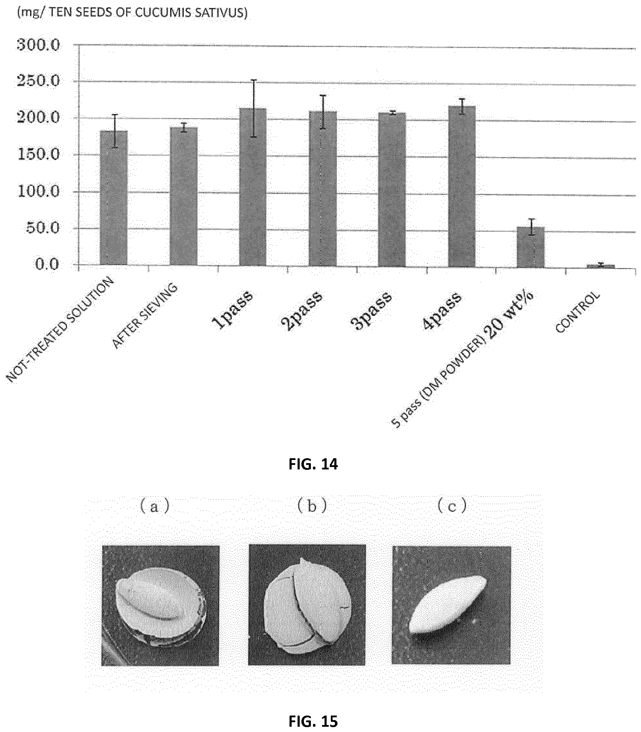

Thereafter, the seeds of cucumis sativus coated with Towada stone powder were naturally dried at room temperature about for two days, and then were weighed. A difference between this weight and the weight of the seeds of cucumis sativus was used as a coating amount of Towada stone powder. A test was performed three times continuously in each test group. Results thereof are illustrated in Fig. 14.

Each of the not-treated suspension, the sieved suspension, and the pulverized (1 to 4 pass) suspension exhibited a high coating amount.

It is considered that the high coating amount exhibited by the not-treated suspension is caused by a fact that Towada stone powder in the Towada stone powder suspension filtered in the third tank of the overflow type precipitation container is already equalized before the sieving treatment according to the sieving step and the pulverizing treatment according to the pulverizing step are performed (Fig. 12(b)) and the particle size distribution thereof converges in a range of 0.25 µm to 1.10 µm (Fig. 13(a)).

In addition, it was confirmed that the coating amount of the Towada stone powder suspension in the third tank of the precipitation container was clearly higher than the Towada stone (DMpowder) powder suspension obtained by readjusting the concentration of the suspension pulverized (5 pass) under 160 Mpa using the powdery Towada stone (DM powder) described above as a raw material to 20 wt% (Fig. 14).

For reference, coated seeds of cucumis sativus obtained by coating the seeds of cucumis sativus with the Towada stone powder suspension (20 wt%) in the third tank of the precipitation container, pulverized through the four stages as described above, are illustrated in Fig. 15.

It was confirmed that these seeds were more sufficiently coated than the seeds of cucumis sativus coated with the Towada stone (DM powder) powder suspension (20 wt%) (Fig. 15(a)) (Fig. 15(b)).

According to these results, it was confirmed that the Towada stone powder suspension pulverized such that the average particle diameter of Towada stone powder converges in a range of 0.9 µm to 2.6 µm and the mode value in a particle size distribution converges in a range around 0.39 µm (0.25 µm to 1.10 µm) was suitable for a coating material for seeds.

Particularly, it was confirmed that a coating material for seeds could be prepared only through a step of naturally filtering Towada stone powder.

Therefore, it is possible to provide a coating material for seeds obtained through a simple treatment step not requiring a spreading agent, an organic protective agent, a decompression treatment, or the like unlike prior art, and a coated seed coated with an appropriate amount of the material, suffering from little physical damage, and almost free from growth inhibition.

### (Exemplary experiment 2)

An experiment for examining an application of natural inorganic minerals used as an agricultural material and having different hardnesses to a coating material for seeds was performed.

### (1) Seed coating test using a natural mineral other than Towada stone

A seed coating test was performed using an inorganic mineral powder pulverized through two stages as described above for each of zeolite (Mohs hardness 5) and bentonite (Mohs hardness 1 to 2).

Towada stone has a Vickers hardness of 710, which is 6 to 7 in terms of Mohs hardness.

In this exemplary experiment, seeds of cucumis sativus (product name: Shimoshirazuj ibai manufactured by Tohoku Seed Co., Ltd.) were used as seeds to be coated. An appropriate amount of the inorganic mineral powder suspension pulverized through two stages as described above was dropwise added to the seeds of cucumis sativus.

Thereafter, the resulting seeds were dried to obtain coated seeds of cucumis sativus coated with the inorganic mineral powder. The coating amount of the inorganic mineral powder in the coated seeds was measured by the following procedures.

Ten seeds of cucumis sativus were weighed (average weight: 260.4 mg, standard deviation: 10.0 mg). Thereafter, each of the following inorganic mineral powder suspensions pulverized through two stages as described above was dropwise added to the seeds of cucumis sativus at 200 µL/seed.

- zeolite powder suspension having a particle diameter of 800 µm or less (20 wt%)
- zeolite powder suspension having a particle diameter of 100 µm or less (20 wt%)
- bentonite powder suspension having a particle diameter of 300 to 500 µm (20 wt%)
- bentonite powder suspension having an average particle diameter of 70 µm (20 wt%)

Thereafter, the seeds of cucumis sativus coated with each of the inorganic mineral powders were naturally dried at room temperature about for 19 hours, and then were weighed. A difference between each of these weights and the weight of the seeds of cucumis sativus was used as a coating amount of inorganic mineral powder. A test was performed four times continuously in each test group. Results thereof are illustrated in Fig. 16.

As illustrated in Fig. 16, it was confirmed that the coating amount of the seeds having a smaller particle diameter was larger in each of zeolite and bentonite.

As illustrated in Fig. 17, it was confirmed that the seeds were coated sufficiently in each of zeolite and bentonite.

According to this result, it was confirmed that an inorganic mineral powder suspension having an average particle diameter of 70 µm to 100 µm was also suitable for a coating material for seeds.

### (2) Seed coating test using seeds of glycine max (green soybeans)

In this exemplary experiment, seeds of glycine max (green soybeans) (product name: Waseedamame manufactured by Tohoku Seed Co., Ltd.) were used as seeds to be coated. An appropriate amount of the Towada stone powder suspension in the third tank of the precipitation container in each step of the pulverizing treatment was dropwise added to the seeds of glycine max (green soybeans).

Thereafter, the resulting seeds were dried to obtain coated seed of glycine max (green soybeans) coated with Towada stone powder. The coating amount of Towada stone powder in the coated seeds was measured by the following procedures.

Ten seeds of glycine max (green soybeans) were weighed (average weight: 4129.2 mg, standard deviation: 258.5 mg). Thereafter, the Towada stone powder suspension (20 wt%) in the third tank of the precipitation container in each step of the pulverizing treatment was dropwise added to the seeds of glycine max (green soybeans) on a plastic petri dish at 400 µL/seed. Among the steps of the pulverizing treatment, the pulverizing step was performed through four stages (4 pass).

The DM powder suspension (20 wt%) was dropwise added to the seeds of glycine max (green soybeans) on a plastic petri dish at 400 µL/seed as a control.

Adhesion of the suspension to the entire seeds of glycine max was confirmed by inclining the plastic petri dish. Thereafter, the seeds of glycine max (green soybeans) coated with Towada stone powder were naturally dried at room temperature about for 18 hours, and then were weighed. A difference between this weight and the weight of the seeds of glycine max (green soybeans) was used as a coating amount of Towada stone powder. A test was performed three times continuously in each test group. Results thereof are illustrated in Fig. 18.

As illustrated in Fig. 18, it was confirmed that the coating amount onto the seeds was about 6.8 times that of the DM powder suspension due to pulverizing Towada stone powder as described above.

According to these results, it was confirmed that even an inorganic mineral other than Towada stone couldbe applied to a coating material for seeds. Particularly, it has been suggested that an inorganic mineral powder having a Mohs hardness of 1 to 7 can be applied to a coating material for seeds.

In addition, it was confirmed that a coating material for seeds could be prepared only through a step of pulverizing these inorganic mineral powders in suspensions.

In addition, it was confirmed that the coating amount of an inorganic mineral in a coating material of a coated seed coated with such a coating material for seeds was maintained at a value enough for coating.

Therefore, it is possible to provide a coating material for seeds obtained through a simple treatment step not requiring a spreading agent, an organic protective agent, a decompression treatment, or the like unlike prior art, and a coated seed coated with an appropriate amount of the material, suffering from little physical damage, and almost free from growth inhibition.

Hereinabove, the preferred embodiment of the present invention has been described. However, the present invention is not limited to the above embodiment, and can be modified variously within a technical range understood from description of claims.

### Industrial Applicability

According to the present invention, a coating material for seeds obtained through a simple treatment step and a coated seed coated with this coating material can be obtained. Therefore, the present invention can be applied to a technology for allowing this coating material for seeds to support antagonistic microorganisms. It is possible to provide a coating material for seeds suitable for protecting a crop from disease or insect damage and a coated seed coated with this coating material, suffering from little physical damage, and almost free from growth inhibition.

## Claims

1. A coating material for seeds containing a pulverized inorganic mineral powder.

2. The coating material for seeds according to claim 1, wherein the inorganic mineral powder has a Mohs hardness of 1 to 7.

3. The coating material for seeds according to claim 2, wherein the pulverized inorganic mineral powder has an average particle diameter of 0.9 µm to 100 µm.

4. The coating material for seeds according to claim 3, wherein the pulverized inorganic mineral powder has a mode value of 0.25 µm to 1.10 µm in a particle size distribution.

5. A coating material for seeds, wherein the coating material for seeds according to claim 4 is a liquid composition having a predetermined viscosity.

6. A coated seed coated with the coating material for seeds according to any one of claims 1 to 5.
